# EUROPEAN PATENT SPECIFICATION

(11) **EP 2 792 741 B1**
(45) Date of publication and mention of the grant of the patent: **30.09.2015**
(21) Application number: 13164277.9
(22) Date of filing: 18.04.2013
(51) Int. Cl.: C12N 5/071

(54) **Method for isolation of adipose tissue-derived stromal vascular fraction cells**
Verfahren zur Isolierung von fettgewebeabgeleiteten vaskulären Bindegewebefraktionszellen
Procédé d'isolement de cellules de fraction vasculaire stromale dérivée de tissu adipeux

(43) Date of publication of application: 22.10.2014
(73) Proprietor: Michalek, Jaroslav, 624 00 Brno (CZ)
(72) Inventor: Michalek, Jaroslav, 624 00 Brno (CZ)
(74) Representative: Hartvichova, Katerina

(56) References cited:
- CN-B- 101 693 884
- MASSIMO FAUSTINI ET AL: "Nonexpanded Mesenchymal Stem Cells for Regenerative Medicine: Yield in Stromal Vascular Fraction from Adipose Tissues", TISSUE ENGINEERING PART C: METHODS, vol. 16, no. 6, 1 December 2010 (2010-12-01), pages 1515-1521, XP055071049, ISSN: 1937-3384, DOI: 10.1089/ten.tec.2010.0214
- ZUK P A ET AL: "Human adipose tissue is a source of multipotent stem cells", MOLECULAR BIOLOGY OF THE CELL, AMERICAN SOCIETY FOR CELL BIOLOGY, US, vol. 13, no. 12, 20 December 2002 (2002-12-20), pages 4279-4295, XP002249630, ISSN: 1059-1524, DOI: 10.1091/MBC.E02-02-0105
- ASTORI GIUSEPPE ET AL: "In vitro and multicolor phenotypic characterization of cell subpopulations identified in fresh human adipose tissue stromal vascular fraction and in the derived mesenchymal stem cells", JOURNAL OF TRANSLATIONAL MEDICINE, BIOMED CENTRAL, LONDON, GB, vol. 5, no. 1, 31 October 2007 (2007-10-31), page 55, XP021037638, ISSN: 1479-5876
- TAHA M F ET AL: "Isolation, identification and multipotential differentiation of mouse adipose tissue-derived stem cells", TISSUE AND CELL, CHURCHILL LIVINGSTONE MEDICAL JOURNALS, EDINBURGH, GB, vol. 42, no. 4, 1 August 2010 (2010-08-01) , pages 211-216, XP027223936, ISSN: 0040-8166 [retrieved on 2010-05-21]
- TALENS-VISCONTI ET AL: "Human mesenchymal stem cells from adipose tissue: Differentiation into hepatic lineage", TOXICOLOGY IN VITRO, ELSEVIER SCIENCE, GB, vol. 21, no. 2, 7 February 2007 (2007-02-07), pages 324-329, XP005878144, ISSN: 0887-2333, DOI: 10.1016/J.TIV.2006.08.009
- BIANCA GALATEANU ET AL: "Layer-shaped alginate hydrogels enhance the biological performance of human adipose-derived stem cells", BMC BIOTECHNOLOGY, BIOMED CENTRAL LTD. LONDON, GB, vol. 12, no. 1, 29 June 2012 (2012-06-29), page 35, XP021126584, ISSN: 1472-6750, DOI: 10.1186/1472-6750-12-35

## Description

### Field of Invention

The present invention provides an improved method for isolation of adipose tissue-derived stromal vascular fraction cells (SVF). Specifically, it provides an improved method for enrichment of SVF and enhancement of its therapeutic effects.

### Background Art

Stem cells have been shown to repopulate and repair tissues, organs and/or organ systems. Thus, they offer an enormous therapeutic potential. Moreover, stem cells have the capability to regenerate themselves, which is the important characteristic to maintain themselves in the body as a vital source for regenerating cells and tissues.

Multipotent mesenchymal stem cells are located in many areas of the body. Accumulating evidence supports the existence of stem cells in a number of postnatal organs and connective tissues. Besides bone marrow, they have been isolated from periosteum (Choi IS et al., Multipotency and growth characteristic of periosteum-derived progenitor cells for chondrogenic, osteogenic, and adipogenic differentiation. Biotechnol Lett, 2008, 30: 593-60), synovial membrane (De Bari C et al., Multipotent mesenchymal stem cells from adult human synovial membrane. Arthritis Rheum, 2001, 44:1928-1942), skeletal muscle (Dodson MV et al., Skeletal muscle stem cells from animals I. Basic cell biology. Int J Biol Sci, 2010, 6: 465-474), skin (Belicchi M et al., Human skin-derived stem cells migrate throughout forebrain and differentiate into astrocytes after injection into adult mouse brain. J Neurosci Res, 2004, 77:475-486), pericytes (Feng J et al., Perivascular cells as mesenchymal stem cells. Expert Opin Biol Ther, 2010, 10:1441-1451), deciduous teeth (Miura M et al., Shed: Stem cells from human exfoliated deciduous teeth. Proc Natl Acad Sci USA, 2003, 100:5807-5812) or umbicilal cord (Baksh D et al., Comparison of proliferative and multilineage differentiation potential of human mesenchymal stem cells derived from umbilical cord and bone marrow. Stem Cells, 2007, 25:1384-1392). Although stem cells from these sources are valuable, only a limited amount of tissue is available and thus, only a low number of cells can be harvested. On the contrary, stem cells derived from white adipose tissue, termed adipose tissue-derived stem cells (ASCs), exhibit several advantageous features (Zuk PA et al., Human adipose tissue is a source of multipotent stem cells. Mol Biol Cell, 2002; Tobita M et al., Adipose-derived stem cells: Current findings and future perspectives. Discov Med, 2011, 11:160-170).

Adipose tissue is accessible, abundant and replenishable, thereby providing a potential adult stem cell reservoir for each individual. Furthermore, it can be obtained in large quantities with only little patient discomfort. Moreover, adipose tissue is derived from the mesenchyme and contains a supportive stroma that can be easily isolated. Adipose tissue is composed of two different cell populations, mature adipose cells, which constitute more than 90 % of the tissue volume and the SVF. The SVF cells are heterogeneous with respect to expression of classical hematopoietic markers (Mitchell J et al., The immunophenotyppe of human adipose derived cells: Temporal changes in stromal- and stem cell-associated markers (Stem Cells, 2006, 24(2): 376-385) and include several cell types, such as ASCs, preadipocytes, endothelial precursor cells, smooth muscle cells, fibroblasts, lymphocytes, pericytes, monocytes and macrophages (Zuk PA et al., Human adipose tissue is a source of multipotent stem cells. Mol Biol Cell, 2002, 13(12): 4279-95; Han J et al., Adipose tissue is an extramedullary reservoir for functional hematopoietic stem and progenitor cells. Blood, 2010, 115: 957-964). The SVF cells appear to have multiple mesodermal lineage capabilities *in vitro.* After exposure to the appropriate differentiating environment, ASCs are able to differentiate into angiogenic (Rehman J et al., Secretion of angiogenic and antiapoptotic factors by human adipose stromal cells. Circulation, 2004, 109:1292-1298), cardiomyogenic (Lee WC et al., Cardiomyogenic differentiation potential of human adipose precursor cells. Int J Cardiol, 2009, 133:399-401; Planat-Benard V et al., Spontaneous cardiomyocyte differentiation from adipose tissue stroma cells. Circ Res, 2004, 94:223-229), neurogenic (Safford KM et al., Characterization of neuronal/glial differentiation of murine adipose-derived adult stromal cells. Exp Neurol, 2004, 187:319-328), myogenic (Mizuno H et al., Myogenic differentiation by human processed lipoaspirate cells. Plast Reconstr Surg, 2002, 109:199-209), osteogenic (Dragoo JL et al., Tissue-engineered cartilage and bone using stem cells from human infrapatellar fat pads. J Bone Joint Surg Br, 2003, 85:740-747), adipogenic (Cherubino M and Marra KG, Adipose-derived stem cells for soft tissue reconstruction. Regen Med, 2009, 4:109-117; Rubin JP and Marra KG, Soft tissue reconstruction. Methods Mol Biol, 2011, 702:395-400), chondrogenic (Estes BT et al., Isolation of adipose-derived stem cells and their induction to a chondrogenic phenotype. Nat Protoc, 2010, 5:1294-1311; Estes BT and Guilak F, Three-dimensional culture systems to induce chondrogenesis of adipose-derived stem cells. Methods Mol Biol, 2011, 702:201-217), periodontogenic (Tobita M and Mizuno H, Periodontal disease and periodontal tissue regeneration. Curr Stem Cell Res Ther, 2010, 5:168-174) lineages and other cell populations with specialized functions. Taken together, adipose tissue represents a very valuable source of stem cells.

Although stem cells are not able to move on their own through the body directly to a damaged location, by means of a single medical procedure it can be beneficial to isolate stem cells from another source and transfer them to the desired destination in the body. Subsequently, through their self-regulatory actions, the stem cells are able to regenerate and renew damaged tissues at the damaged location and thus, can be used in treatment of numerous diseases (Zuk PA et al., Multilineage cells from human adipose tissue: Implications for cell-based therapies. Tissue Eng, 2001, 7:211-228; Gimble JM et al., Adipose-derived stem cells for regenerative medicine. Circ Res, 2007, 100:1249-1260; Utsunomiya T et al., Human adipose-derived stem cells: potential clinical applications in surgery. Surg Today, 2011, 41(1):18-23; Lindroos B et al., The potential of adipose stem cells in regenerative medicine. Stem Cell Rev and Rep, 2011, 7:269-291; Cherubino M et al., Adipose-derived stem cells for wound healing applications. Ann Plast Surg, 2011, 66:210-215; Mizuno H et al., Concise review: Adipose-derived stem cells as a novel tool for future regenerative medicine. Stem Cells, 2012, 30:804-810). This effect can be used in orthopedics to treat arthritis, in bone and cartilage repair, in regenerative medicine for treating ulcers, chronic wounds and burns, in cardiovascular tissue regeneration, ischemia revascularization as well as in plastic surgery, for example for breast augmentation without the need to use foreign material of any kind.

Typically, the adipose tissue aspirate is obtained from abdominal subcutaneous fat of adipose tissue. Using liposuction, a requisite amount of adipose tissue can be safely removed from the patient. The amount of tissue collected depends on numerous factors, including the body mass index, age of the donor, the availability of accessible adipose tissue harvest sites, concomitant and pre-existing medications and conditions, and the clinical purpose for which the tissue is being collected. Then, stem cells are isolated from the extracted adipose tissue. Subsequently, these cells can be used for therapeutic and regenerative purposes.

Recently, SVF cells were obtained by a method described by Zuk (Zuk PA et al., Human adipose tissue is a source of multipotent stem cells. Mol Biol Cell, 2002). Briefly, lipoaspirated fat is massively washed with normal saline, followed by digestion of adipose tissue with collagenase and calcium chloride that serves as collagenase activator. SVF cells are released from adipose tissue, typically after 30-60 min. incubation of lipoaspirate with collagenase and calcium chloride at 37°C. The suspension is then centrifuged and SVF cells are pelleted and collected for further experimental or clinical use. To minimize tissue debris contamination of SVF cells that are typically 10-50 µm in diameter, cells are passed through 60-100 µm filter and harvested for further application, ie. cell culture or direct clinical administration. Unfortunately, this approach is rather time consuming and number of cells that can be harvested is limited. The aim of the present invention is to improve the method of SVF cell isolation and enrichment.

### Disclosure of the Invention

The present invention provides a method of isolation of adipose tissue-derived stromal vascular fraction cells (SVF cells), wherein adipose tissue is washed using sterile normal saline solution in order to remove remaining blood, local anesthetics and other impurities. The washed adipose tissue is then resuspended in normal saline supplemented with sterile grade of collagenase and calcium gluconate. The suspension is then incubated for 10 to 60 minutes at 36 to 39 °C and subsequently centrifuged at 400 to 500g. The upper layer, containing mature adipocytes, is removed. Normal saline solution is added to the pellet containing SVF to form a suspension. The SVF is then passed through a filter to remove remaining larger tissue pieces.

Adipose tissue can be obtained from the body of a patient by numerous methods known to a person of ordinary skill in the art.

Calcium gluconate is preferably used in the concentration range of 0.1 to 5 mmol/l, more preferably at about 1 mmol/l.

The incubation with calcium gluconate is preferably carried out at 37 °C for 15 minutes. Preferably, the suspension is incubated while shaking at 100 to 500 rpm, more preferably at 250 rpm.

The centrifugation is preferably carried out for 5 minutes, more preferably at 430 rpm.

Preferably, the filter has a pore size of 60 to 150 µm, more preferably 100 µm.

The present invention provides an improved method of adipose tissue-derived SVF isolation leading to higher yields of stem cells using calcium gluconate, and a safe and contaminant-free process of isolation of SVF from adipose tissue.

### Example of carrying out the Invention

From male and female subjects undergoing therapeutic procedures, subcutaneous adipose tissue sites are obtained by lipoaspiration. The obtained adipose tissue is washed using sterile normal saline solution in order to remove remaining blood and local anestetics, used for lipoaspiration, from the adipose tissue. Lipoaspirate is then resuspended in normal saline supplemented with sterile grade of collagenase and calcium gluconate or CaCl₂. Calcium ions are used at 1.0 mmol/l concentration and the reaction is carried out at 37°C for 15 minutes at 37°C, while shaking at 250 rpm. The reaction product is then centrifuged at 430g for 5 minutes. Subsequently, the upper layer of lipid, containing mature adipocytes, is removed. Normal saline solution is added to the pellet containing SVF to form a suspension. The SVF is then passed through the filter with 60-150 µm, ideally 100 µm, pores to remove remaining larger tissue pieces.

Comparison of SVF isolation from adipose tissue with CaCl₂ or with calcium gluconate was performed. The processing of 10 samples, each containing 50 ml of freshly obtained adipose tissue by lipoaspiration, was performed as described above and CaCl₂ or calcium gluconate at 1.0 mmol/l concentration was used. As a result, the amount of nucleated cells obtained in SVF was significantly higher when calcium gluconate was added, ie. 735,000 ± 118,000 (with calcium gluconate) versus 629,000 ± 147,000 (with CaCl₂) SVF viable cells per ml of adipose tissue (p<0.05). Viability was comparable in both arms of isolated SVF cells, ie. 94.6 ± 6.7% with calcium gluconate and 93.8 ± 9.4% with CaCl₂. When all of these cells were harvested and further tested for sterility (standard 7-day Bact-alert test), none of tested samples demonstrated bacterial contamination.

## Claims

1. A method of isolation of adipose tissue-derived stromal vascular fraction cells (SVF cells), wherein adipose tissue is washed using sterile normal saline solution, the washed adipose tissue is then resuspended in normal saline supplemented with sterile grade of collagenase and calcium gluconate, the suspension is then incubated for 10 to 60 minutes at 36 to 39 °C and subsequently centrifuged at 400 to 500g, after centrifugation the upper layer is removed and the normal saline solution is added to the pellet containing SVF cells to form a suspension, which is then passed through a filter to remove remaining larger tissue pieces.

2. The method of claim 1, wherein calcium gluconate is used in the concentration range of 0.1 to 5 mmol/l.

3. The method of any of the preceding claims, wherein the incubation with calcium gluconate is carried out at 37 °C for 15 minutes.

4. The method of any of the preceding claims, wherein the incubation with calcium gluconate is carried out while shaking at 100 to 500 rpm.

5. The method of any of the preceding claims, wherein the centrifugation is carried out for 5 minutes.

6. The method of any of the preceding claims, wherein the filter has a pore size of 60 to 150 µm.

## Patentansprüche

1. Verfahren zur Isolierung von fettgewebeabgeleiteten vaskulären Bindegewebefraktionszellen (SVF), wobei das Fettgewebe mit steriler physiologischer Lösung gewaschen wird, das gewaschene Fettgewebe danach in einer mit steriler Kollagenase und Kalziumglukonat ergänzten physiologischen Lösung resuspendiert wird, diese Suspension danach für die Dauer von 10 bis 60 Minuten bei 36 bis 39 °C inkubiert und nachfolgend bei 400 bis 500g zentrifugiert wird, nach dem Zentrifugieren die Oberschicht beseitigt und zu dem die SVF-Zellen enthaltenden Pellet die physiologische Lösung unter Bildung von Suspension zugegeben wird, die nachfolgend zur Beseitigung grösserer Gewebepartikeln über ein Filter filtriert wird.

2. Verfahren nach dem Anspruch 1, wobei das Kalziumglukonat im Konzentrationsbereich 0,1 bis 5 mmol/l verwendet wird.

3. Verfahren nach einem der vorhergehenden Ansprüche, wobei die Inkubation mit Kalziumglukonat bei 37 °C für die Dauer von 15 Minuten durchgeführt wird.

4. Verfahren nach einem der vorhergehenden Ansprüche, wobei die Inkubation mit Kalziumglukonat während des Schüttelns bei 100 bis 500 Drehungen/Minute durchgeführt wird.

5. Verfahren nach einem der vorhergehenden Ansprüche, wobei das Zentrifugieren für die Dauer von 5 Minuten durchgeführt wird.

6. Verfahren nach einem der vorhergehenden Ansprüche, wobei das Filter eine Porengrösse von 60 bis 150 µm aufweist.

## Revendications

1. Procédé d'isolement de cellules de fraction vasculaire stromale (cellules SVF) dérivée du tissu adipeux, le tissu adipeux étant lavé à l'aide d'une solution physiologique stérile, le tissu adipeux lavé étant ensuite remis en suspension dans une solution physiologique supplémentée de collagénase stérile et de gluconate de calcium, la suspension étant ensuite incubée pendant 10 à 60 minutes à la température de 36 à 39 °C et ultérieurement centrifugée de 400 à 500g, après centrifugation la couche supérieure est éliminée et la solution physiologique est ajoutée au culot contenant les cellules SVF pour former une suspension, qui est ensuite passée à travers un filtre pour éliminer les pièces de tissu plus grandes restantes.

2. Procédé selon la revendication 1, **caractérisé en ce que** le gluconate de calcium est utilisé dans l'intervalle de concentration de 0.1 à 5 mmol/l.

3. Procédé selon l'une quelconque des revendications précédentes, **caractérisé en ce que** l'incubation avec le gluconate de calcium est effectuée à 37 °C pendant 15 minutes.

4. Procédé selon l'une quelconque des revendications précédentes, **caractérisé en ce que** l'incubation avec le gluconate de calcium est effectuée durant l'agitation de 100 à 500 tr / min.

5. Procédé selon l'une quelconque des revendications précédentes, **caractérisé en ce que** la centrifugation est effectuée pendant 5 minutes.

6. Procédé selon l'une quelconque des revendications précédentes, **caractérisé en ce que** la taille des pores du filtre est de 60 à 150 µm.
